# EUROPEAN PATENT SPECIFICATION

(11) **EP 4 370 200 B1**
(45) Date of publication and mention of the grant of the patent: **05.03.2025**
(21) Application number: 22751317.3
(22) Date of filing: 13.07.2022
(51) Int. Cl.: A61N 1/378, A61N 1/372

(54) **IMPEDANCE TOMOGRAPHY SYSTEM FOR ALIGNMENT FEEDBACK OF A CHARGING DEVICE FOR AN IMPLANTABLE MEDICAL DEVICE**
IMPEDANZTOMOGRAFIESYSTEM ZUR AUSRICHTUNGSRÜCKKOPPLUNG EINER LADEVORRICHTUNG FÜR EINE IMPLANTIERBARE MEDIZINISCHE VORRICHTUNG
SYSTÈME DE TOMOGRAPHIE PAR IMPÉDANCE PERMETTANT UN RETOUR D'ALIGNEMENT D'UN DISPOSITIF DE CHARGE POUR UN DISPOSITIF MÉDICAL IMPLANTABLE

(30) Priority: 14.07.2021 US 202163221526 P; 10.08.2021 DE 202021104268 U
(43) Date of publication of application: 22.05.2024
(73) Proprietor: BIOTRONIK SE & Co. KG, 12359 Berlin (DE)
(72) Inventor: MCINTOSH, David, Wilsonville, Oregon 97070 (US)
(74) Representative: Biotronik Corporate Services SE
(86) International application number: PCT/EP2022/069540
(87) International publication number: WO 2023/285503

(56) References cited:
- WO-A1-2009/055579
- WO-A1-2010/042054
- US-A1- 2005 187 590
- US-A1- 2015 174 415

## Description

The present invention relates to a medical system comprising an implantable medical device and an external charging device for charging an energy storage device of the medical device, for example in the form of an implantable pulse generator (IPG).

In a receiver for a resonant inductive system for wireless power transfer (WPT), a coil at the charging device excites a coil at the implantable medical device. The alignment of the coils on the charging device and the implantable medical device is critical for highly efficient energy transfer. Efficient energy transfer allows for less thermal stress on the patient and potentially faster charging times. Alignment feedback is also important for the patient to quickly align the charging device over the implantable or implanted medical device (in some cases in awkward locations such as the back).

One way to determine alignment is to identify the conductive metallic titanium housings typically used in implants (pacemakers, neurostimulators).

Impedance tomography is a technique that measures the impedance of a target object to infer the composition or presence of a material. Impedance measurements consist of an array of electrodes (at least two) to which an alternating current is output and the corresponding voltage is measured. According to Ohm's law, impedance is Z=V/I, where V is the measured complex voltage and I is the injected complex current. Complex refers to the resistive and reactive components of the impedance. Based on the medium in which the current flows, the impedance changes. Impedance tomography is sensitive to electrical conductivity. In this regard, larger electrode arrays allow for finer resolution imaging.

In the prior art, arrays of magnetic field sensor coils are known to be located on the coil of the charging device. When the charging device is over a metal target, the magnetic field sensor coils change amplitude. Furthermore, a detection of the primary coil of the charging device (metal detector mode) is known. Furthermore, the presence of a backscatter (e.g. LSK) can also be detected.

Further, US 2018/0212451 A1 discloses charging an energy source located in an implantable medical device implanted in a patient, the charging device comprising first and second pairs of electric coils configured to generate first and second uniform magnetic fields in overlapping first and second cylindrical regions located between the respective pairs of electric coils.

Further, WO 2019/173866 A1 discloses a device for providing sensory stimulation to a subject, the device comprising a coil system having at least one coil and an electronic controller operating in accordance with software instructions. In use, the controller receives input signals, performs an analysis of the input signals, and uses the results of the analysis to cause a signal generator to generate stimulation signals, the stimulation signals being applied to the coil system to thereby generate a stimulating electromagnetic field in a target region of the subject. Document US-A-2015/174415 discloses the most relevant prior art.

Based on the prior art set forth above, the present invention is directed to implementing a means for providing rapid alignment feedback to a patient attempting to position an external charging device over their implant for optimal and efficient energy transfer.

This task is solved by a medical system having the features of claim 1. Advantageous embodiments of the invention are described below. The invention is defined in claim 1.

According to claim 1, a medical system is disclosed, comprising:
- an implantable medical device comprising an energy storage device (e.g., in the form of a battery) for supplying electrical energy to the implantable medical device, and a secondary coil for transferring electrical energy to the energy storage device, and
- an external or separate charging device designed for charging the energy storage device, the charging device comprising a primary coil via which electrical energy can be transferred to the energy storage device via the secondary coil of the implantable medical device.

According to the invention, it is provided that the charging device has a plurality of metal electrodes which are arranged in front of the primary coil and are each assigned to a segment of a housing surface of the charging device, the segments forming a two-dimensional field or array, and the charging device being designed to use the metal electrodes to calculate an impedance for each segment in order to check whether the primary coil is aligned with respect to the secondary coil for optimum energy transfer, i.e., is opposite the latter, in particular coaxially.

The system according to the invention thus enables the detection of the position of the implanted medical device and thus the possibility for the charging device to provide alignment feedback (direction and rough distance) to the patient. The invention thus promotes efficient energy transfer from the charging device to the implant. In other words, the charging device has an impedance tomography function to identify the position of the implant (e.g., IPG) and then provide alignment feedback to the user between the coils on both sides.

According to a preferred embodiment of the medical system according to the present invention, the charging device comprises at least two metal electrodes arranged on the housing surface and adapted to contact the skin of a patient.

Further, according to a preferred embodiment of the medical system according to the present invention, the housing surface is formed by a flat substrate connected to a rest of the charging device.

According to a preferred embodiment, the flat substrate is a patch or a plaster.

Further, according to a preferred embodiment of the medical system according to the present invention, the energy storing device is a battery.

Further, according to a preferred embodiment of the medical system according to the present invention, the charging device is adapted to induce an alternating current in the metal electrodes and to measure the corresponding alternating voltage induced by this current, wherein the charging device is further adapted to calculate the complex impedance (Z=V/I) from the alternating current I and the corresponding alternating voltage V for at least four segments 32, particularly for 16 segments of the housing surface.

Further, according to a preferred embodiment of the medical system according to the present invention, on the basis of images which indicate the impedances in the individual segments, a presence of conductors and non-conductors is distinguished by the medical system on the basis of the impedances to determine an orientation of the primary and secondary coils with respect to one another.

Further, according to a preferred embodiment of the medical system according to the present invention, the charging device is adapted to output user feedback for the purpose of aligning the primary coil over the secondary coil.

Further, according to a preferred embodiment of the medical system according to the present invention, the medical system is adapted to provide the user feedback via a hand-held remote control or via warning tones.

According to yet another aspect of the present disclosure, which is not part of the invention, a medical system is disclosed, the medical system comprising:
- an implantable medical device comprising an energy storage device for supplying electrical energy to the medical device and a secondary coil for transferring electrical energy to the energy storage device, and
- a charging device which is designed to charge the energy storage device, the charging device comprising multiple primary coils in an array, wherein the medical system is adapted to use impedance tomography to determine which primary coil is optimal for activation to achieve high power transfer efficiency.

In the following, embodiments of the present invention and other features and advantages of the invention will be explained with reference to the Figures, wherein
- Fig. 1: shows a housing surface of a charging device of a system according to the invention, the housing surface comprising an array of segments, each segment having an associated metal electrode arranged in front of a primary coil of the charging device,
- Fig. 2: shows a schematic sectional view of the charging device and implantable medical device (implanted in a patient), wherein the metal electrodes on the housing surface contact the skin surface of the patient,
- Fig. 3: shows the segments of the housing surface, although here there is still no optimal alignment of the charging device because the axes of the primary and secondary coils do not coincide, and
- Fig. 4: shows an optimal alignment of the charging device with the coil axes of the primary and secondary coils coinciding.

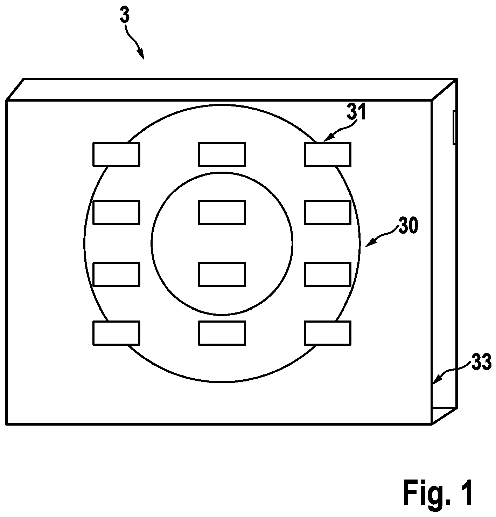

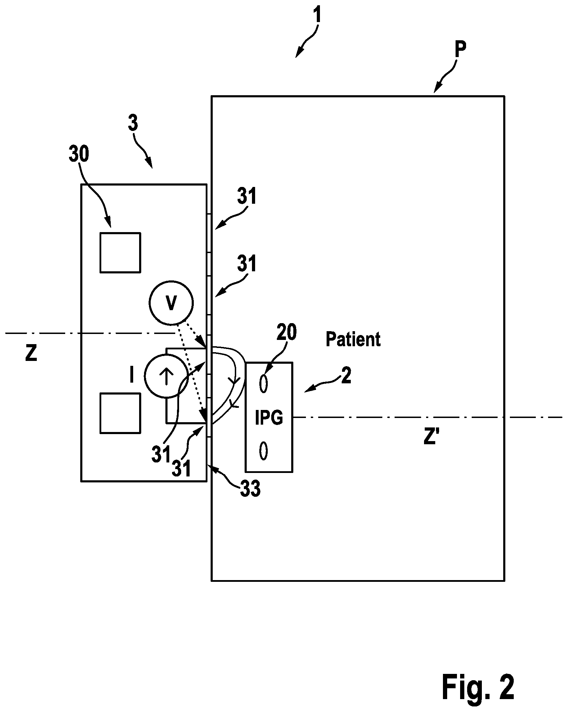

Figure 1 shows, in connection with Figure 2, a medical system 1 according to the invention with a wireless power transfer (WPT) system comprising an external charging device 3 and an implantable medical device 2 (e.g. IPG), wherein the charging device 3 includes a primary coil 30, and wherein the implantable medical device 2 implanted in a patient P according to Figure 2 includes a secondary coil 20.

The charging device 3 includes at least two metal electrodes 31 proximate to the body and adapted to contact the skin of the patient P. The electrodes 31 are arranged on a housing surface 33 of the charging device 3. The housing surface 33 may also be formed by a flat substrate (e.g. patch or plaster) suitably connected to the rest of the charging device 3.

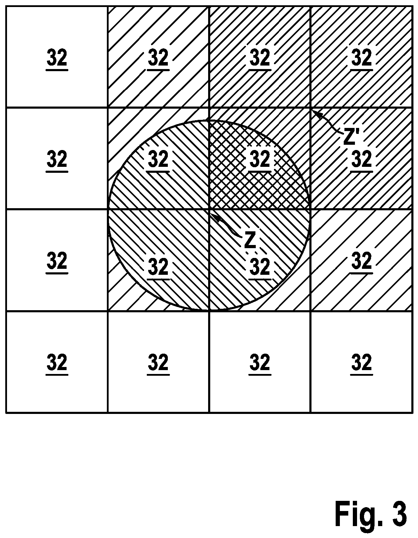

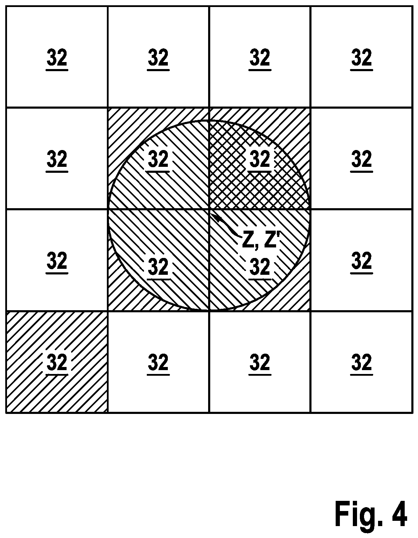

Referring to Figure 2, the charging device 3 is adapted to induce an alternating current I in the metal electrodes 31, the charging device 3 measuring the corresponding alternating voltage V induced by this current. From this, the charging device 3 calculates the complex impedance (Z=V/I) for at least four segments 32 or, in this case, for all segments 32 of the housing surface 33 shown in Figures 3 and 4. On the basis of these images, which indicate the impedances in the individual segments 32 and which are shown by way of example in Figures 3 and 4 for various orientations of the coils 20, 30 on both sides, the presence of conductors and non-conductors is distinguished on the basis of the impedances, so that it is possible to determine the orientation of the coils 20, 30 with respect to one another. Preferably, the charging device 3 is adapted to output user feedback for the purpose of aligning the charging device 3 or the primary coil 30 over the metallic implant 2 or its secondary coil 20. The user feedback can be provided, for example, via a hand-held remote control, via warning tones or the like.

Figure 3 shows an example of an impedance image that results from calculating the impedances for the segments 32. In Figure 3, the Z' axis of the secondary coil 20 is too far up and to the right of the center or Z axis of the primary coil 30.

After alignment feedback by the charging device 3 to the patient P and patient movement of the charging device 3, the positioning changes to the optimal alignment shown in Figure 4, where the coil axes Z, Z' coincide.

Another embodiment of the disclosure, which is not part of the invention, features a charging device with multiple primary coils in an array. Impedance tomography is used to determine which coil is optimal for activation to achieve high power transfer efficiency. This is the opposite of the single primary coil moving to find the implant; here the correct coil is selected electronically.

The disclosure allows reliable identification of implant 2 compared to other methods that use multiple sense/search coils to look for disturbances in the excitation field. Impedance tomography may also prove useful for other reasons (e.g., to determine tissue response to the implant, particularly inflammation, scar tissue, etc.).

## Claims

1. A medical system (1), comprising:
- an implantable medical device (2) comprising an energy storage device for supplying electrical energy to the medical device (2) and a secondary coil (20) for transferring electrical energy to the energy storage device,
- a charging device (3) which is designed to charge the energy storage device, the charging device having a primary coil (30) via which electrical energy can be transferred to the energy storage device via the secondary coil (20) of the implantable medical device (2),
**characterized in that**
the charging device (3) comprises a plurality of metal electrodes (31) which are arranged in front of the primary coil (30) and are each assigned to a segment (32) of a housing surface (33) of the charging device (3), the charging device (3) being designed to use the metal electrodes (31) to calculate an impedance for each segment (32) and to use the impedances to check whether the primary coil (30) is aligned for optimum energy transfer to the secondary coil (20).

2. The medical system according to claim 1, wherein the charging device (3) comprises at least two metal electrodes (31) arranged on the housing surface (33) and adapted to contact the skin of a patient (P)

3. The medical system according to claim 1 or 2, wherein the housing surface (33) is formed by a flat substrate connected to a rest of the charging device (3).

4. The medical system according to claim 3, wherein the flat substrate is a patch or a plaster.

5. The medical system according to one of the preceding claims, wherein the energy storing device is a battery.

6. The medical system according to one of the preceding claims, wherein the charging device (3) is adapted to induce an alternating current (I) in the metal electrodes (31) and to measure the corresponding alternating voltage (V) induced by this current, wherein the charging device (3) is further adapted to calculate the complex impedance (Z=V/I) from the alternating current (I) and the corresponding alternating voltage (V) for at least four segments (32) or for 16 segments (32) of the housing surface (33).

7. The medical system according to claim 6, wherein on the basis of images which indicate the impedances in the individual segments (32) a presence of conductors and non-conductors is distinguished by the medical system on the basis of the impedances to determine an orientation of the primary and secondary coils (20, 30) with respect to one another.

8. The medical system according to one of the preceding claims, wherein the charging device (3) is adapted to output user feedback for the purpose of aligning the primary coil (30) over the secondary coil (20).

9. The medical system according to claim 8, wherein the medical system is adapted to provide the user feedback via a hand-held remote control or via warning tones.

## Patentansprüche

1. Medizinisches System (1), umfassend:
- eine implantierbare medizinische Vorrichtung (2), die eine Energiespeichervorrichtung zum Liefern von elektrischer Energie zu der medizinischen Vorrichtung (2) und eine Sekundärspule (20) zum Übertragen von elektrischer Energie auf die Energiespeichervorrichtung umfasst,
- eine Ladevorrichtung (3), die dazu eingerichtet ist, die Energiespeichervorrichtung zu laden, wobei die Ladevorrichtung eine Primärspule (30) aufweist, über die elektrische Energie über die Sekundärspule (20) der implantierbaren medizinische Vorrichtung (2) auf die Energiespeichervorrichtung übertragen werden kann,
**dadurch gekennzeichnet, dass**
die Ladevorrichtung (3) eine Mehrzahl von vor der Primärspule (30) angeordneten Metallelektroden (31) umfasst, die jeweils einem Segment (32) einer Gehäuseoberfläche (33) der Ladevorrichtung (3) zugeordnet sind, wobei die Ladevorrichtung (3) dazu eingerichtet ist, unter Verwendung der Metallelektroden (31) eine Impedanz für jedes Segment (32) zu berechnen und unter Verwendung der Impedanz zu prüfen, ob die Primärspule (30) für eine optimale Energieübertragung auf die Sekundärspule (20) ausgerichtet ist.

2. Medizinisches System nach Anspruch 1, wobei die Ladevorrichtung (3) mindestens zwei an der Gehäuseoberfläche (33) angeordnete Metallelektroden (31) umfasst, die für einen Kontakt mit der Haut eines Patienten (P) ausgelegt sind.

3. Medizinisches System nach Anspruch 1 oder 2, wobei die Gehäuseoberfläche (33) von einem flachen Substrat gebildet wird, das mit einem Rest der Ladevorrichtung (3) verbunden ist.

4. Medizinisches System nach Anspruch 3, wobei das flache Substrat ein Patch oder Pflaster ist.

5. Medizinisches System nach einem der vorangehenden Ansprüche, wobei die Energiespeichervorrichtung eine Batterie ist.

6. Medizinisches System nach einem der vorangehenden Ansprüche, wobei die Ladevorrichtung (3) dafür ausgelegt ist, einen Wechselstrom (I) in den Metallelektroden (31) zu induzieren und die entsprechende, von diesem Strom induzierte Wechselspannung (V) zu messen, wobei die Ladevorrichtung (3) ferner dafür ausgelegt ist, für mindestens vier Segmente (32) oder für 16 Segmente (32) der Gehäuseoberfläche (33) die komplexe Impedanz (Z=V/I) aus dem Wechselstrom (I) und der entsprechenden Wechselspannung (V) zu berechnen.

7. Medizinisches System nach Anspruch 6, wobei auf Basis von Bildern, welche die Impedanz in den individuellen Segmenten (32) angeben, von dem medizinischen System auf der Basis der Impedanzen ein Vorhandensein von Leitern und Nichtleitern erkannt wird, um eine gegenseitige Ausrichtung der Primär- und der Sekundärspule (20, 30) zu bestimmen.

8. Medizinisches System nach einem der vorangehenden Ansprüche, wobei die Ladevorrichtung (3) dafür eingerichtet ist, für den Zweck der Ausrichtung der Primärspule (30) über der Sekundärspule (20) eine Benutzerrückmeldung auszugeben.

9. Medizinisches System nach Anspruch 8, wobei das medizinische System dafür ausgelegt ist, die Benutzerrückmeldung über eine tragbare Fernsteuerung oder über Warntöne bereitzustellen.

## Revendications

1. Système médical (1), comprenant :
- un dispositif médical implantable (2) comprenant un dispositif de stockage d'énergie destiné à fournir de l'énergie électrique au dispositif médical (2) et une bobine secondaire (20) destinée à transférer l'énergie électrique au dispositif de stockage d'énergie,
- un dispositif de chargement (3) qui est conçu pour charger le dispositif de stockage d'énergie, le dispositif de chargement ayant une bobine primaire (30) par l'intermédiaire de laquelle l'énergie électrique peut être transférée au dispositif de stockage d'énergie par l'intermédiaire de la bobine secondaire (20) du dispositif médical implantable (2),
**caractérisé en ce que**
le dispositif de chargement (3) comprend une pluralité d'électrodes métalliques (31) qui sont agencées devant la bobine primaire (30) et sont chacune attribuée à un segment (32) d'une surface de boîtier (33) du dispositif de chargement (3), le dispositif de chargement (3) étant conçu pour utiliser les électrodes métalliques (31) afin de calculer une impédance pour chaque segment (32) et pour utiliser les impédances afin de contrôler si la bobine primaire (30) est alignée pour un transfert d'énergie optimal sur la bobine secondaire (20).

2. Système médical selon la revendication 1, dans lequel le dispositif de chargement (3) comprend au moins deux électrodes métalliques (31) agencées sur la surface de boîtier (33) et adaptées pour entrer en contact avec la peau d'un patient (P)

3. Système médical selon la revendication 1 ou 2, dans lequel la surface de boîtier (33) est formée par un substrat plat connecté à un reste du dispositif de chargement (3).

4. Système médical selon la revendication 3, dans lequel le substrat plat est un timbre ou un plâtre.

5. Système médical selon l'une des revendications précédentes, dans lequel le dispositif de stockage d'énergie est une batterie.

6. Système médical selon l'une des revendications précédentes, dans lequel le dispositif de chargement (3) est adapté pour induire un courant alternatif (I) dans les électrodes métalliques (31) et pour mesurer la tension alternative (V) correspondante induite par ce courant, dans lequel le dispositif de chargement (3) est en outre adapté pour calculer l'impédance complexe (Z = V/I) à partir du courant alternatif (I) et de la tension alternative (V) correspondante pour au moins quatre segments (32) ou pour 16 segments (32) de la surface de boîtier (33).

7. Système médical selon la revendication 6, dans lequel sur la base des images qui indiquent les impédances dans les segments individuels (32) une présence de conducteurs et de non conducteurs est distinguée par le système médical sur la base des impédances pour déterminer une orientation des bobines primaire et secondaire (20, 30) l'une par rapport à l'autre.

8. Système médical selon l'une des revendications précédentes, dans lequel le dispositif de chargement (3) est adapté pour renvoyer un retour utilisateur dans le but d'aligner la bobine primaire (30) sur la bobine secondaire (20).

9. Système médical selon la revendication 8, dans lequel le système médical est adapté pour fournir le retour utilisateur par l'intermédiaire d'une commande à distance portative ou par l'intermédiaire de signaux sonores d'avertissement.
